# EUROPEAN PATENT SPECIFICATION

(11) **EP 3 998 935 B1**
(45) Date of publication and mention of the grant of the patent: **15.11.2023**
(21) Application number: 20786051.1
(22) Date of filing: 21.09.2020
(51) Int. Cl.: A61B 5/00

(54) **WIRE MANAGEMENT COUPLER FOR A MEDICAL DEVICE**
KABELVERWALTUNGSKOPPLER FÜR EINE MEDIZINISCHE VORRICHTUNG
COUPLEUR DE GESTION DE FIL POUR UN DISPOSITIF MÉDICAL

(30) Priority: 20.09.2019 US 201962903410 P
(43) Date of publication of application: 25.05.2022
(73) Proprietor: St. Jude Medical International Holding S.à r.l., 2449 Luxembourg (LU)
(72) Inventor: STROM, Nicholas, Minneapolis, Minnesota 55413 (US); HELGESON, Zachary L., Richfield, Minnesota 55423 (US); WAKEFIELD, Jodee, Minneapolis, Minnesota 55408 (US); TEGG, Troy T., Elk River, Minnesota 55330 (US)
(74) Representative: Alpspitz IP
(86) International application number: PCT/IB2020/058807
(87) International publication number: WO 2021/053648

(56) References cited:
- WO-A1-2017/177121
- WO-A1-2017/192712

## Description

### a. Field of the Disclosure

This disclosure relates to a wire management coupler for a medical device.

### b. Background Art

Catheters have been used for cardiac medical procedures for many years. Catheters can be used, for example, to diagnose and treat cardiac arrhythmias, while positioned at a specific location within a body that is otherwise inaccessible without a more invasive procedure. A prior art catheter is disclosed in patent publication WO2017/192712 A1.

Mapping catheters may include, for example, a diagnostic sensor and/or a therapeutic device disposed on a distal end of the catheter. Each one of the diagnostic sensor and/or therapeutic devices can be electrically coupled to one or more controllers (e.g., computing devices), which can send and/or receive signals to and/or from the diagnostic sensor and/or therapeutic device. The wires that electrically couple the diagnostic sensor and/or the therapeutic device to the one or more controllers can oftentimes be greater than 40 gauge, which can result in the wires being fragile.

The diagnostic sensor and/or therapeutic device can be disposed at a distal end of a catheter shaft and their associated wiring can be routed along the catheter shaft. To enable the catheter shaft to be threaded through a tortuous vasculature of a patient, the shaft oftentimes needs to have a corresponding flexibility. As a result of the regular bending of the catheter shaft, care must be taken when routing the wires associated with the diagnostic sensor and/or therapeutic device along the catheter shaft.

The foregoing discussion is intended only to illustrate the present field and should not be taken as a disavowal of claim scope.

### BRIEF SUMMARY

The invention is best summarized by the appended claims.

### BRIEF DESCRIPTION OF THE DRAWINGS

Fig. 1A depicts a top view of a high density electrode catheter, according to various embodiments of the present disclosure.
Fig. 1B depicts an isometric side and top view of the high density electrode catheter depicted in Fig. 1A, according to various embodiments of the present disclosure.
Fig. 2A depicts an isometric view of a coupler that can be disposed in a medical device, according to various embodiments of the present disclosure.
Fig. 2B depicts the coupler of Fig. 2A without the first and second magnetic position sensors, in accordance with embodiments of the present disclosure.
Fig. 2C depicts a cross-sectional side view of a proximal end of the coupler, depicted in Fig. 2B, along the elongate coupler axis AA, in a direction of arrow BB, in accordance with embodiments of the present disclosure.
Fig. 2D depicts a proximal end view of coupler, in accordance with embodiments of the present disclosure.
Fig. 3A depicts a cross-sectional side view of a proximal end of a coupler along the elongate coupler axis A'A', similar to that depicted in Fig. 2C, but with an additional wire management feature, in accordance with embodiments of the present disclosure.
Fig. 3B depicts a cross-sectional side view of a proximal end of a coupler along the elongate coupler axis A"A", similar to that depicted in Fig. 3A, but with a wire management feature of an alternative design, in accordance with embodiments of the present disclosure.
Fig. 4 depicts a coupler that includes an alternate wire routing, in accordance with embodiments of the present disclosure.

### DETAILED DESCRIPTION

Catheters can generally be used to perform diagnostic and/or therapeutic procedures on patients. For example, electrophysiology catheters can include various sensors and/or therapeutic devices, including magnetic sensors, thermocouples, electrodes, or other electrically based sensing transducers. Wires can be used to connect these devices with electrophysiology systems, which provide physicians and/or clinicians with electro-anatomical data and/or control over the various sensors and devices. The wires providing the data and/or control over the various devices can be subjected to stresses during typical catheter use, including but not limited to, insertion/withdrawal of the catheter in an introducer or other sheath, deflection of the distal end of the device, and various other maneuvers or manipulations inside of the patient's vasculature or cardiac anatomy. It is beneficial to design a medical device in a manner to avoid excessive stresses on associated wires that would compromise signals being passed through them.

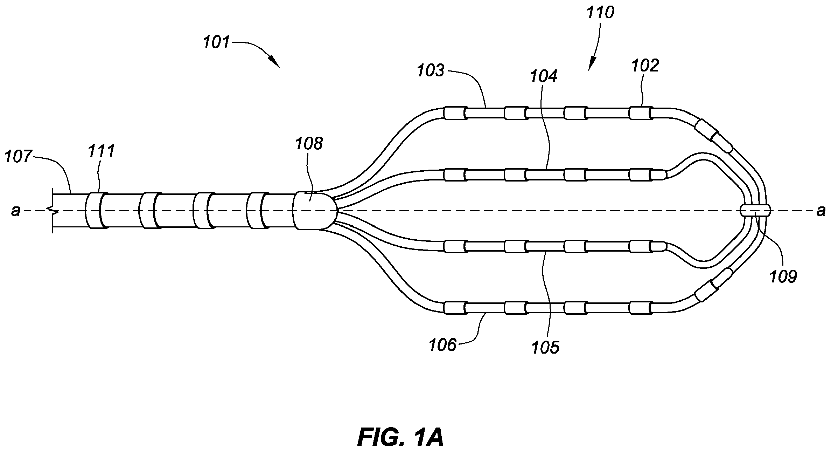

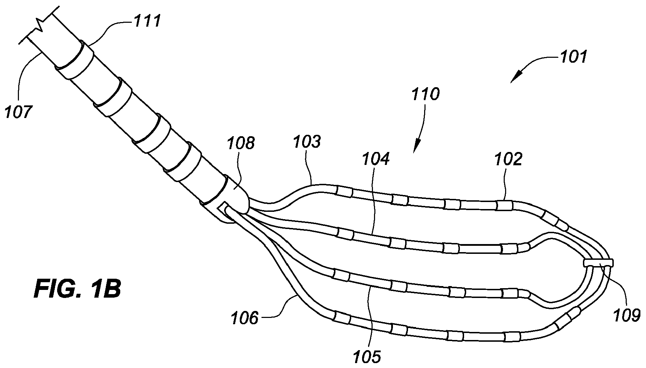

Fig. 1A is a top view of a high density electrode catheter 101 and Fig. 1B is an isometric side and top view of the high density electrode catheter 101, according to various embodiments of the present disclosure. In some embodiments, the high density electrode catheter 101 can include a flexible tip portion 110 that forms a flexible array of microelectrodes 102. This planar array (or 'paddle' configuration) of microelectrodes 102 comprises four side-by-side, longitudinally-extending arms 103, 104, 105, 106, which can form a flexible framework on which the microelectrodes 102 are disposed. The four microelectrode-carrier arms can comprise a first outboard arm 103, a second outboard arm 106, a first inboard arm 104, and a second inboard arm 105, which can be joined at a distal end by a distal connective portion 209. These arms can be laterally separated from each other.

Each of the four arms can carry a plurality of microelectrodes 102. For example, each of the four arms can carry microelectrodes 102 spaced along a length of each of the four arms. Although each of the high density electrode catheters 101 depicted in Figs. 1A and 1B depict four arms, the high density electrode catheters 101 could comprise more or fewer arms. Additionally, while the high density electrode catheter 101 depicted in Figs. 1A and 1B depict 18 electrodes (e.g., 5 microelectrodes on the first outboard arm 103 and second outboard arm 106 and 4 microelectrodes on the first inboard arm 104 and second inboard arm 105), the catheters can include more or fewer than 18 electrodes. In addition, the first outboard arm 103 and second outboard arm 106 can include more or fewer than 5 microelectrodes and the first inboard arm 104 and second inboard arm 105 can include more or fewer than 4 microelectrodes).

In some embodiments, the microelectrodes 102 can be used in diagnostic, therapeutic, and/or mapping procedures. For example and without limitation, the microelectrodes 102 can be used for electrophysiological studies, pacing, cardiac mapping, and ablation. In some embodiments, the microelectrodes 102 can be used to perform unipolar or bipolar ablation. This unipolar or bipolar ablation can create specific lines or patterns of lesions. In some embodiments, the microelectrodes 102 can receive electrical signals from the heart, which can be used for electrophysiological studies. In some embodiments, the microelectrodes 102 can perform a location or position sensing function related to cardiac mapping.

In some embodiments, the high density electrode catheter 101 can include a catheter shaft 107. The catheter shaft 107 can include a proximal end and a distal end. The distal end can include a connector 108, which can couple the distal end of the catheter shaft 107 to a proximal end of the planar array. The catheter shaft 107 can define a catheter shaft longitudinal axis aa, as depicted in Fig. 1A, along which the first outboard arm 103, first inboard arm 104, second inboard arm 105, and second outboard arm 106 can generally extend parallel in relation therewith. The catheter shaft 107 can be made of a flexible material, such that it can be threaded through a tortuous vasculature of a patient. In some embodiments, the catheter shaft 107 can include one or more ring electrodes 111 disposed along a length of the catheter shaft 107. The ring electrodes 111 can be used for diagnostic, therapeutic, and/or mapping procedures, in an example.

As depicted in Fig. 1B, the flexible tip portion 110 can be adapted to conform to tissue (e.g., cardiac tissue). For example, when the flexible tip portion 110 contacts tissue, the flexible tip portion 110 can deflect, allowing the flexible framework to conform to the tissue. In some embodiments, the arms (or the understructure of the arms) comprising the paddle structure (or multi-arm, electrode-carrying, flexible framework) at the distal end of the catheters depicted in Figs. 1A and 1B are preferably constructed from a flexible or spring-like material such as Nitinol and/or a flexible substrate, as discussed herein. The construction (including, for example, the length and/or diameter of the arms) and material of the arms can be adjusted or tailored to create, for example, desired resiliency, flexibility, foldability, conformability, and stiffness characteristics, including one or more characteristics that may vary from the proximal end of a single arm to the distal end of that arm, or between or among the plurality of arms comprising a single paddle structure. The foldability of materials such as Nitinol and/or flexible substrate provide the additional advantage of facilitating insertion of the paddle structure into a delivery catheter or introducer, whether during delivery of the catheter into the body or removal of the catheter from the body at the end of a procedure.

Among other things, the disclosed catheters, with their plurality of microelectrodes, are useful to (1) define regional propagation maps of particularly sized areas (e.g., one centimeter square areas) within the atrial walls of the heart; (2) identify complex fractionated atrial electrograms for ablation; (3) identify localized, focal potentials between the microelectrodes for higher electrogram resolution; and/or (4) more precisely target areas for ablation. These mapping catheters and ablation catheters are constructed to conform to, and remain in contact with, cardiac tissue despite potentially erratic cardiac motion. Such enhanced stability of the catheter on a heart wall during cardiac motion provides more accurate mapping and ablation due to sustained tissue-electrode contact. Additionally, the catheters described herein may be useful for epicardial and/or endocardial use. For example, the planar array embodiments depicted herein may be used in an epicardial procedure where the planar array of microelectrodes is positioned between the myocardial surface and the pericardium. Alternatively, the planar array embodiments may be used in an endocardial procedure to quickly sweep and/or analyze the inner surfaces of the myocardium and quickly create high-density maps of the heart tissue's electrical properties.

In some embodiments, use of the high density electrode catheter 101 can sometimes be plagued by coagulation of blood on various portions of the high density electrode catheter 101. For example, coagulation of blood can occur on the flexible tip portion 110 and/or on the connector 108 of the high density electrode catheter 101. Although coagulation of blood is discussed herein, in some instances other material can be collected on the flexible tip portion 110 and/or on the connector 108, such as tissue cells, for example. Coagulation of blood can impair the functionality of the microelectrodes if the blood coagulates on the microelectrodes. Additionally, coagulation of blood on the flexible tip portion 110 and/or on the connector 108 can cause clots to occur, if the coagulated blood breaks free. As such, it can be beneficial to prevent the coagulation of blood and/or accumulation of other material on the flexible tip portion 110 and/or on the connector 108, which can be accomplished through use of embodiments discussed in the present disclosure.

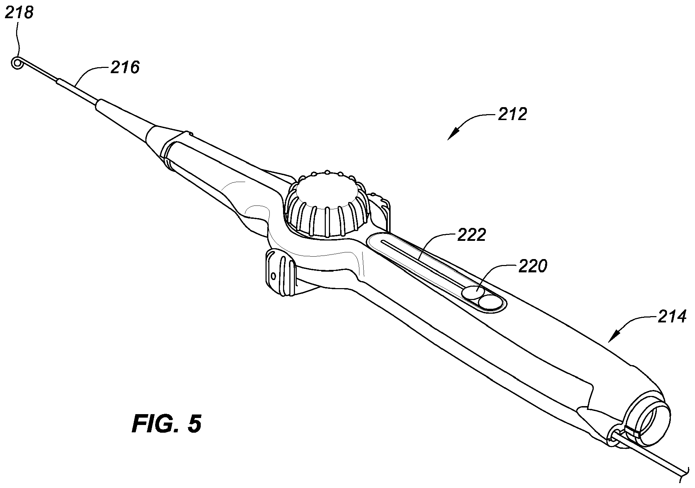

Although some embodiments of the present disclosure include a flexible tip portion that includes diagnostic and/or therapeutic electrodes, embodiments of the present disclosure can include a flexible and/or rigid tip portion (e.g., distal assembly) in lieu of or in addition to the flexible tip portion, which can be an electrode assembly or any number of end use therapeutic and/or diagnostic devices. For example, the tip portion can include an ultrasound sensor and/or transducer, such as that associated with an intracardiac echocardiography (ICE) catheter; a laser, balloon or any other number of therapeutic and/or diagnostic devices. Furthermore, although Figs. 1A and 1B depict a particular configuration of a flexible tip portion 110, embodiments of the present disclosure can be used with other types of medical devices. In an example, embodiments of the present disclosure can be used with medical devices that include other types of flexible tip portions 110, such as a basket catheter, circular mapping catheter, among other types of therapeutic and/or diagnostic medical devices. Another exemplary type of medical device that can employ embodiments of the present disclosure is depicted and discussed in relation to Fig. 5.

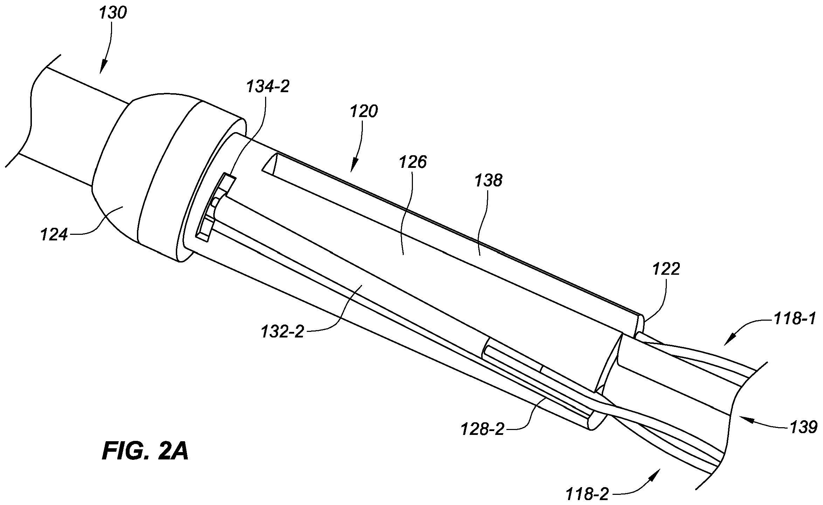

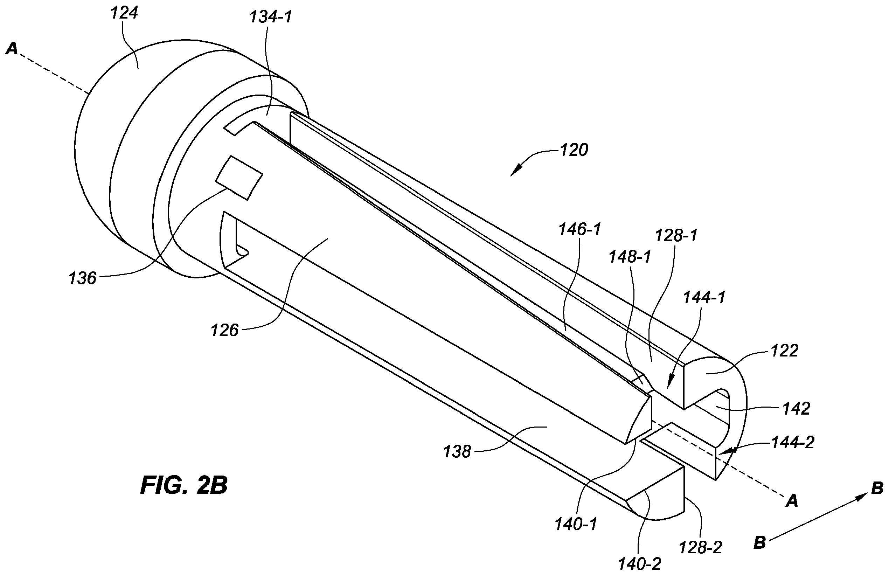

Fig. 2A depicts an isometric view of a coupler 120 that can be disposed in a medical device, according to various embodiments of the present disclosure. In some embodiments, the coupler 120 can define an elongate coupler axis AA (also referred to herein as neutral axis AA), which extends parallel with and through a center of the coupler 120. The coupler 120, in some embodiments, can couple an end effector 130 (e.g., looped distal end, flexible tip portion, etc.) to a distal end of an elongate shaft (e.g., catheter shaft 107 in Figs. 2A and 2B). The coupler 120 can have a proximal end 122 and a distal end 124 and can axially extend along the elongate coupler axis AA (Fig. 2B). In an example, a body portion 126 can be inserted into a distal end of an elongate shaft. In some embodiments, the distal end 124, also referred to herein as coupler head 124, can have a diameter that is greater than the body portion 126 and greater than an inner diameter of the elongate shaft into which the body portion is disposed, such that the distal end 124 prevents the coupler from being inserted too far into the elongate shaft.

In some embodiments, the coupler 120 can define a first sensor groove 128-1 and a second sensor groove 128-2 (Fig. 12C) in which a first sensor 132-1, hidden from view, and a second sensor 132-2 can be disposed. As further discussed in PCT Application No. US2017/030828, which is hereby incorporated by reference as though fully set forth herein, the first sensor groove 128-1 and the second sensor groove 128-2 can be formed at angles with respect to one another and the first sensor 132-1 and the second sensor 132-2 can be disposed within the first sensor groove 128-1 and the second sensor groove 128-2, such that the first sensor 132-1 and the second sensor 132-2 are disposed at angles with respect to one another. As further depicted, a plurality of wires 118-1, 118-2 can extend from a distal end of the coupler 120. As discussed, the wires can be electrically coupled with one or more sensors 132-1, 132-2, in some embodiments.

Placement slots (e.g., first placement slot 134-1 depicted in Fig. 2B, second placement slot 134-2) can be defined in an outer surface of the body portion 126 of the coupler 120. A placement slot can be located at the distal end of each sensor groove 128-1, 128-2. For example, with respect to the first sensor groove 128-1, the first placement slot 134-1 (Fig. 2B) can be located at the distal end of the first sensor groove 128-1. In some embodiments, as depicted, the first placement slot 134-1 can be a cross-longitudinal slot defined in the exterior surface of the body portion 126. In an example, the placement slots can extend perpendicular to each one of the sensor grooves 128-1, 128-2. For instance, as depicted with respect to the second placement slot 134-2, the second placement slot 134-2 can extend perpendicular to the second sensor groove 128-2 and the distal portion of the sensor 132-2 can be disposed in the placement slot 134-2 for visual verification that the sensor 132-2 has been correctly placed in the second placement slot 134-2.

Fig. 2B depicts the coupler of Fig. 2A without the first and second magnetic position sensors, in accordance with embodiments of the present disclosure. As depicted, the coupler 120 can extend along an elongate coupler axis AA. The coupler 120 can have a proximal end 122 and a distal end 124 and can axially extend along the elongate coupler axis AA. In an example, a body portion 126 can be inserted into a distal end of an elongate shaft. In some embodiments, the distal end 124 can have a diameter that is greater than the body portion 126 and greater than an inner diameter of the elongate shaft into which the body portion is disposed, such that the distal end 124 prevents the coupler from being inserted too far into the elongate shaft.

The coupler 120 can define first and second sensor grooves 128-1, 128-2, which can extend along sensor axes, which are divergent with respect to the elongate coupler axis. In some embodiments, sensors (e.g., magnetic position sensors) can be disposed in the first and second sensor grooves 128-1, 128-2.

In some embodiments, the body portion 126 can define one or more holes (e.g., first hole 136) in the body portion 126. In some embodiments, the holes 136 can be formed in an exterior surface of the body portion 126 and can provide a location for adhesive to collect when the body portion is disposed within a distal end of an elongate shaft.

In some embodiments, a longitudinally extending channel 138 can extend through a portion of the body portion 126. In an example, as depicted, the longitudinally extending channel 138 can be defined by a pair of inner side walls 140-1, 140-2 and an inner top wall 142. As depicted, the pair of inner side walls 140-1, 140-2 can be parallel in some embodiments, although not required. For example, the pair of inner sidewalls 140-1, 140-2 can be divergent in some embodiments, forming a flared channel where a distance between the pair of inner sidewalls 140-1, 140-2 narrows towards the inner top wall 142 and/or forming a contracted channel where a distance between the pair of inner sidewalls 140-1, 140-2 increases towards the inner top wall 142. In some embodiments, the longitudinally extending channel 138 can provide space for one or more tubes, wires, etc., which can extend through the longitudinally extending channel 138 and out the proximal end 122 of the coupler 120. In some embodiments, the tubes 139 (Fig. 2A), wires, etc. that extend through the channel 138 can fluidly, electrically, and/or mechanically couple one or more features included in the end effector 130. Furthermore, the longitudinally extending channel 138 can house other components associated with a medical device, although not mentioned herein.

According to the invention, the first sensor groove 128-1 and the second sensor groove 128-2 include a wire management feature located at a proximal end of each one of the first sensor groove and the second sensor groove. In some embodiments, the wire management features discussed herein can assist in management of the wires that extend from the proximal end of the coupler 120. For example, the wire management features can manage wires that are electrically coupled to the one or more sensors, or other devices, disposed in the first and second sensor grooves 128-1, 128-2. The wire management features can allow for the wires that exit the proximal end of the sensor grooves 128-1, 128-2 to be located closer to a neutral axis AA of the coupler 120 at the proximal end of the coupler 120. By locating the wires closer to the neutral axis AA of the coupler, the wires can experience less strain when the elongate shaft, into which the coupler 120 is inserted, is deflected. For example, if the wires are not located close to the neutral axis AA of the coupler 120 at the proximal end of the coupler 120, then the wires can experience greater strain at the proximal end of the coupler 120 when the elongate shaft, into which the coupler 120 is inserted, is deflected.

In some embodiments, the wire management feature can include a cutout that is defined in a proximal end 122 of the coupler 120. As depicted in Fig. 2B, the coupler can define a cutout 144-1, 144-2 for each of the respective first and second sensor grooves 128-1, 128-2. In an example, the first and second cutouts 144-1, 144-2 can extend distally from the proximal end 122 of the coupler 120. In some embodiments, the cutouts 1442-1, 144-2 can be defined in the inner sidewalls 140-1, 140-2, coupling the longitudinally extending channel 138 with each one of the first and second sensor grooves 128-1, 128-2.

In some embodiments, the first and second sensor grooves 128-1, 128-2 can have a respective base portion, as illustrated by base portion 146-1. For example, the base portion 146-1 can form a bottom of the sensor groove 128-1. The base portion 146-2 of the second sensor groove 128-2 is hidden from view. Using the first senor groove 128-1 as an example, a sensor can be disposed within the first sensor groove 128-1, as discussed in Fig. 2A. The sensor can be held in place by the opposing walls of the first sensor groove 128-1, as well as the base portion 146-1 of the first sensor groove 128-1.

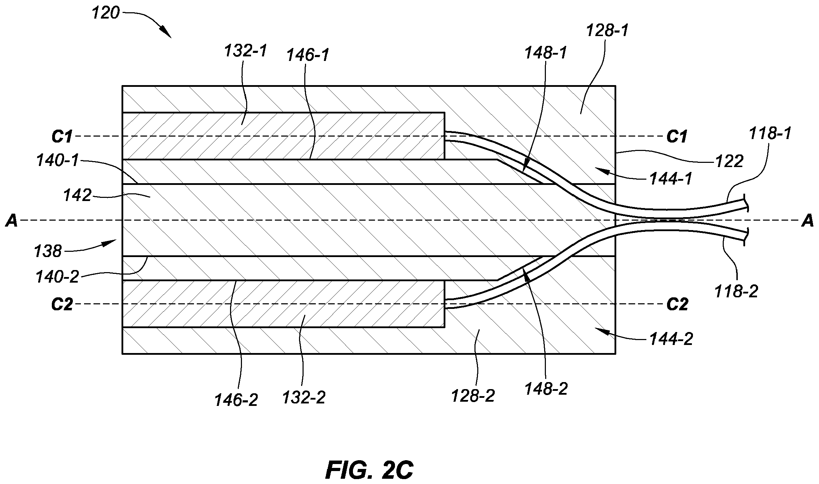

As further depicted with respect to Fig. 2B, in some embodiments, the proximal end of the base portion 146-1, 146-2 (Fig. 2C) can be removed adjacent the proximal end 122 of the coupler, defining cutouts 144-1, 144-2. For example, the proximal end 122 can define recessed areas that extend distally along each one of the sensor grooves 128-1, 128-2, thus forming the cutouts 144-1, 144-2. This can allow for wires associated with the respective sensors disposed in the first and second sensor grooves 128-1, 128-2 to pass through the cutouts 144-1, 144-2 and be disposed closer to a neutral axis AA of the coupler 120.

In the invention a proximal end of the base portion 146-1, 146-2 includes beveled proximal edges 148-1, 148-2 (depicted in Fig. 2C) to better enable a transition for wires contained in each one of the first and second sensor grooves 128-1, 128-2 through the cutouts 144-1, 144-2 towards the neutral axis AA. In an example, the beveled proximal edges 148-1, 148-2 of the proximal ends of the base portion 146-1, 146-2 can be beveled towards the coupler longitudinal axis AA (neutral axis AA) in a distal to proximal direction. In some embodiments, the beveled proximal edges 148-1, 148-2 can include a straight bevel, which is depicted. However, in some embodiments, the beveled proximal edges 148-1, 148-2 can include a radiused edge to provide a smooth transition for the wires towards the neutral axis AA.

Fig. 2C depicts a cross-sectional side view of a proximal end of the coupler 120 along the elongate coupler axis AA, in a direction of arrow BB, in accordance with embodiments of the present disclosure. Fig. 2C further illustrates the features discussed and depicted in relation to Figs. 2A and 2B. For example, the proximal end 122 of the coupler 120 is depicted with wires 118-1, 118-2 extending from the sensors 132-1, 132-2 out the proximal end 122 of the coupler 120. As previously discussed, the sensors 132-1, 132-2 can de disposed in first and second sensor grooves 128-1, 128-2, such that the sensors 132-1, 132-2 are disposed adjacent to base portions 146-1, 146-2, which define a base of the first and second sensor grooves 128-1, 128-2. The coupler further includes the longitudinally extending channel 138, which is defined by the inner side walls 140-1, 140-2, as well as the inner top wall 142.

As depicted, the wires 118-1, 118-2 can extend proximally from each sensor 132-1, 132-2 and towards the neutral axis AA. This can allow for less strain to be introduced to the wires 118-1, 118-2 when the coupler 120 and/or an elongate shaft in which the coupler 120 is disposed is deflected. For example, if the coupler did not include the cutouts 144-1, 144-2, the wires would extend proximally from the proximal ends of the sensors 132-1, 132-2, generally parallel to the neutral axis AA, until they exited the proximal end 122 of the coupler. Thus, the wires 118-1, 118-2 would be spaced apart from one another where they exit the proximal end of the coupler 120. For example, for illustration, the wires 118-1, 118-2 would extend proximally from the sensors 132-1, 132-2, along the sensor axes C1C1 and C2C2, until exiting the proximal end 122 of the coupler 120. As the elongate shaft in which the coupler 120 is disposed deflects, a greater strain would be applied to the wires extending along the sensor axes C1C1 and C2C2, due to their location, which would be spaced apart from the neutral axis AA.

In contrast, embodiments of the present disclosure can include a coupler 120 with cutouts 144-1, 144-2, as described herein. As depicted, the wires 118-1, 118-2 can extend proximally from the proximal ends of the sensors 132-1, 132-2, towards the neutral axis AA, while still being disposed within the confines of the coupler 120. As such, when the wires 118-1, 118-2 exit the confines of the coupler 120 at the proximal end 122, the wires 118-1, 118-2 can generally be disposed along the neutral axis AA, reducing an amount of strain experienced by the wires where they exit the proximal end 122 of the coupler 120. In some embodiments, the beveled proximal edges 148-1, 148-2 can enable a transition for wires contained in each one of the first and second sensor grooves 128-1, 128-2 through the cutouts 144-1, 144-2 towards the neutral axis AA. For example, the beveled proximal edges 148-1, 148-2 can generally be beveled towards the neutral axis AA, thus guiding the wires 118-1, 118-2 towards the neutral axis AA, preventing the wires 118-1, 118-2 from making a sharp transition (e.g., 90 degree bend) towards the neutral axis AA. A sharp transition can occur where no beveled proximal edges 148-1, 148-2 exist. For example, the ends of the base portions 146-1, 146-2 are 90 degree ends (e.g., perpendicular with the neutral axis AA). In such a case, the wires 118-1, 118-2 can contact sharp edges of the proximal ends of the base portions 146-1, 146-2, creating additional stress points on the wires 118-1, 118-2, where the sharp edges contact the wires 118-1, 118-2.

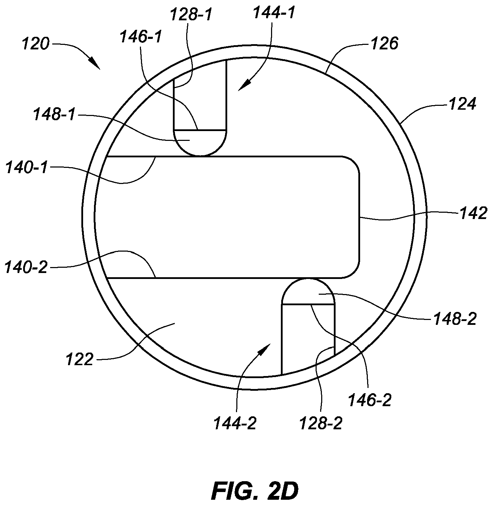

Fig. 2D depicts a proximal end view of coupler 120, in accordance with embodiments of the present disclosure. As depicted, the cutouts 144-1, 144-2 can be defined in the proximal end 122 of the coupler 120, allowing for wires (not depicted) to transition from the sensor grooves 128-1, 128-2 towards the neutral axis. The proximal ends of the base portions 146-1, 146-2 can include beveled proximal edges 148-1, 148-2 to help with the transition between the base portions 146-1, 146-2 to the neutral axis. As depicted, in some embodiments, the beveled proximal edges 148-1, 148-2 can have a concave bevel to better cradle the wires passing through the cutouts 144-1, 144-2 and over the beveled proximal edges 148-1, 148-2. However, the proximal edges 148-1, 148-2 can have any type of bevel, including a straight bevel.

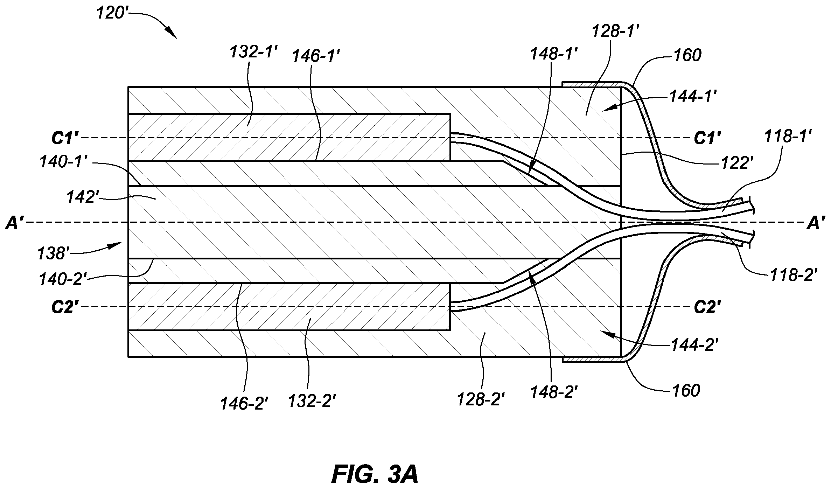

Fig. 3A depicts a cross-sectional side view of a proximal end of a coupler 120' along the elongate coupler axis A'A', similar to that depicted in Fig. 2C, but with an additional wire management feature 160, in accordance with embodiments of the present disclosure. The embodiments depicted in Fig. 3A can include the same and/or similar features as those discussed in relation to Figs. 2A to 2D, as denoted by the addition of a "prime symbol" to the element numbers. For example, Fig. 3A depicts a coupler 120', which is depicted as a coupler 120 in Figs. 2A to 2D. As depicted in Fig. 3A, the wire management feature 160 can include a heat shrink material (e.g., tubing) that can be disposed over a proximal end 122' of the coupler 120'. In some embodiments, a pre-shrunk diameter of the heat shrink material can be larger than the diameter of the proximal end 122' of the coupler 120'. Accordingly, in some embodiments, the heat shrink material can be slid over the coupler 120' and/or the wires 118-1', 118-2'. The heat shrink material can be heated, causing the heat shrink material to shrink and engage an outer surface of the proximal end 122' of the coupler 120'. In some embodiments, this can secure the heat shrink material to the proximal end 122' of the coupler 120'. In some embodiments, the heat shrink material can be heated, causing the heat shrink material to shrink around the wires 118-1', 118-2', which can help to urge the wires 118-1', 118-2' towards the neutral axis A'A', as they exit the proximal end 122' of the coupler 120'. A diameter of the heat shrink material can be large enough to allow for adequate space for assembly, yet small enough to apply a significant enough compressive force to ensure the wires remain as close as possible to the neutral axis A'A' when shrunk. Accordingly, the heat shrink material 160 can help to align the wires 118-1', 118-2' with the neutral axis A'A', preventing and/or reducing strain on the wires 118-1', 118-2' when an elongate shaft in which the coupler 120' is disposed, is deflected.

In some embodiments, the wire management feature 160 can be molded and/or machined from a flexible and/or rigid piece of material. In some embodiments, the wire management feature 160 can be flexible and/or rigid. In some embodiments, the wire management feature 160 can be formed from a molded thermoplastic, silicone, or other polymer (e.g., plastic). In some embodiments, the wire management feature 160 can be flexible to allow for movement of a proximal end of the wire management feature 160 when the coupler 120' is deflected. In an example, the wire management feature 160 can effectively form a cap that is disposed over the proximal end 122' of the coupler 120'. In some embodiments, the wire management feature 160 can be created from a metal. In some embodiments, it may be beneficial for the wire management feature 160 to be more rigid, such that it does not bend. The distal end of the wire management feature 160 can generally have a diameter such that it engages with a proximal end 122' of the coupler 120'. In some embodiments, the wire management feature 160 can be adhered to the proximal end 122' of the coupler 120' with an adhesive.

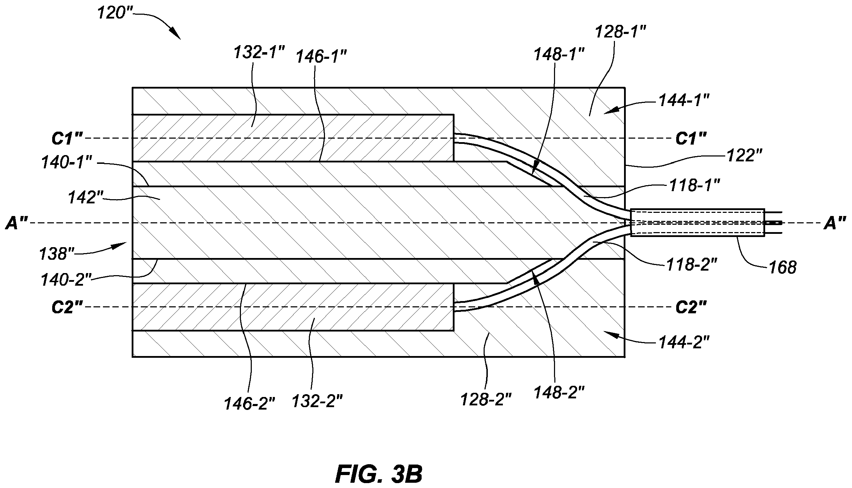

Fig. 3B depicts a cross-sectional side view of a proximal end of a coupler 120" along the elongate coupler axis A"A", similar to that depicted in Fig. 3A, but with a wire management feature 168 of an alternative design, in accordance with embodiments of the present disclosure. The embodiments depicted in Fig. 3B can include the same and/or similar features as those discussed in relation to Figs. 2A to 2D, as denoted by the addition of a "double-prime symbol" to the element numbers. For example, Fig. 3B depicts a coupler 120", which is depicted as a coupler 120 in Figs. 2A to 2D. As depicted in Fig. 3B, the wire management feature 168 can include a tube that can be disposed over the wires 118-1", 118-2" where the wires exit the coupler 120". In some embodiments, the tube can have a diameter that is configured to fit each one of the wires 118-1", 118-2" inside of a lumen defined by the tube 168. As depicted, the tube 168 can be disposed adjacent (e.g., proximally with respect to) a distal end 122" of the coupler 120". In some embodiments, although not depicted, a distal end of the tube 168 can be disposed within the channel 138".

The tube 168 can be formed from a flexible material, allowing the tube 168 to bend when an elongate shaft in which the tube 168 is disposed is deflected. However, in some embodiments, the tube 168 can be formed from a rigid material. In some embodiments, an adhesive can be used to hold the wires 118-1", 118-2 captive within the tube 168. In some embodiments, the tube 168 can be formed from a heat shrink material, which can be heated and shrunk to hold the wires 118-1", 118-2" captive.

In some embodiments, the tube 168 can be connected to the proximal end 122" of the coupler 120". For example, the distal end of the tube 168 can be connected to the proximal end 122" of the coupler 120" within the channel 138". In such an embodiment, the distal end of the tube 168 can extend further into the channel 138" than what is depicted and can be bonded within the channel to the coupler 120".

The tube 168 can help to align the wires 118-1", 118-2" with the neutral axis A"A", preventing and/or reducing strain on the wires 118-1", 118-2" when an elongate shaft in which the coupler 120" is disposed, is deflected. For example, the tube 168 can help to align the wires 118-1", 118-2" with the neutral axis A"A", as they exit the proximal end 122" of the coupler 120". Thus, as the coupler is deflected, the wires 118-1", 118-2" can be subjected to less strain along the neutral axis A"A".

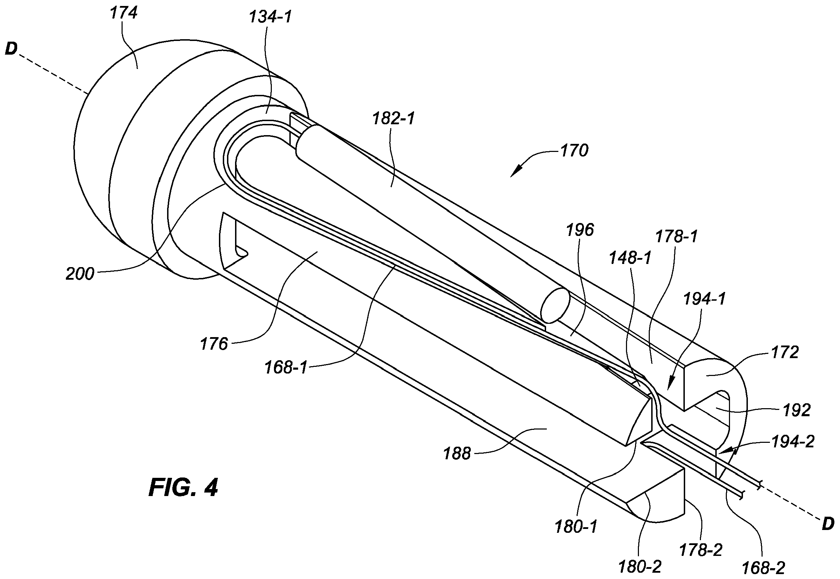

Fig. 4 depicts a coupler 170 that includes an alternate wire routing, in accordance with embodiments of the present disclosure. In some embodiments, a wire management feature can include an alternate wire routing. As depicted, the coupler 170 can define an elongate coupler axis DD, also referred to herein as the neutral axis DD. The coupler 170 can have a proximal end 172 and a distal tip 174 and can axially extend along the elongate coupler axis DD. In an example, a body portion 176 can be inserted into a distal end of an elongate shaft. In some embodiments, the distal end 174 can have a diameter that is greater than the body portion 176 and greater than an inner diameter of the elongate shaft into which the body portion is disposed, such that the distal end 174 prevents the coupler from being inserted too far into the elongate shaft.

The coupler 170 can define first and second sensor grooves 178-1, 178-2, which can extend along sensor axes, which are divergent with respect to the elongate coupler axis. In some embodiments, sensors (e.g., magnetic position sensors) 182-1 can be disposed in the first and second sensor grooves 178-1, 178-2.

In some embodiments, a longitudinally extending channel 188 can extend through a portion of the body portion 176. In an example, as depicted, the longitudinally extending channel 188 can be defined by a pair of inner side walls 190-1, 190-2 and an inner top wall 192. As depicted, the pair of inner side walls 190-1, 190-2 can be parallel in some embodiments, although not required. For example, the pair of inner sidewalls 190-1, 190-2 can be divergent in some embodiments, forming a flared channel where a distance between the pair of inner sidewalls 190-1, 190-2 narrows towards the inner top wall 192 and/or forming a contracted channel where a distance between the pair of inner sidewalls 190-1, 190-2 increases towards the inner top wall 192. In some embodiments, the longitudinally extending channel 188 can provide space for one or more tubes, wires, etc., which can extend through the longitudinally extending channel 188 and out the proximal end 172 of the coupler 170. In some embodiments, the tubes, wires, etc. that extend through the channel 188 can fluidly, electrically, and/or mechanically couple one or more features included in the end effector. Furthermore, the longitudinally extending channel 188 can house other components associated with a medical device, although not mentioned herein.

In some embodiments, the first sensor groove 178-1 and the second sensor groove 178-2 can include a wire management feature located at a proximal end of each one of the first sensor groove and the second sensor groove. In some embodiments, the wire management features discussed herein can assist in management of the wires that extend from the proximal end of the coupler 170. For example, the wire management features can manage wires that are electrically coupled to the one or more sensors, or other devices, disposed in the first and second sensor grooves 178-1, 178-2. The wire management features can allow for the wires that exit the proximal end of the sensor grooves 178-1, 178-2 to be located closer to a neutral axis DD of the coupler 170 at the proximal end of the coupler 170. By locating the wires closer to the neutral axis AA of the coupler, the wires can experience less strain when the elongate shaft, into which the coupler 170 is inserted, is deflected. For example, if the wires are not located close to the neutral axis DD of the coupler 170 at the proximal end of the coupler 170, then the wires can experience greater strain at the proximal end of the coupler 170 when the elongate shaft, into which the coupler 170 is inserted, is deflected.

In some embodiments, the wire management feature can include a cutout that is defined in a proximal end 172 of the coupler 170. As depicted in Fig. 4, the coupler 170 can define a cutout 194-1, 194-2 for each of the respective first and second sensor grooves 178-1, 178-2. In an example, the first and second cutouts 194-1, 194-2 can extend distally from the proximal end 172 of the coupler 170. In some embodiments, the cutouts 194-1, 194-2 can be defined in the inner sidewalls 190-1, 190-2, coupling the longitudinally extending channel 188 with each one of the first and second sensor grooves 178-1, 178-2.

In some embodiments, the first and second sensor grooves 178-1, 178-2 can have a respective base portion, as illustrated by base portion 196. For example, the base portion 196 can form a bottom of the sensor groove 178-1. The base portion of the second sensor groove 178-2 is hidden from view. Using the first senor groove 178-1 as an example, a sensor 182-1 and associated wire 168-2 can be disposed within the first sensor groove 178-1. The sensor can be held in place by the opposing walls of the first sensor groove 178-1, as well as the base portion 196-1 of the first sensor groove 178-1. A second sensor disposed in the second sensor groove 178-2 is hidden from view, although the associated wire 168-2 is depicted.

In contrast to the embodiments depicted in Fig. 2A, the embodiments depicted in Fig. 4 can include an alternate wire routing channel 200, which can extend from a distal end of either one of the sensor grooves 178-1 back in a proximal direction, towards the proximal end 172 of the coupler 170. In some embodiments, as depicted, the alternate wire routing channel 200 can extend from the distal end of the sensor groove 178-1 and can extend proximally, meeting up with a proximal portion of the sensor groove 178-1. In some embodiments, a proximal end of the alternate wire routing channel 200 can be located proximate the proximal end of the sensor 182-1. As depicted, the wire 168-1 can extend from the distal end of the sensor 182-1 through the alternate wire routing channel 200, into the sensor groove 178-1 and out a proximal end 172 of the coupler 170. The wire 168-1 can be routed from the distal end of the sensor 182-1 through the alternate wire routing channel 200 and out of the proximal end 172 of the coupler 170. The alternate wire routing channel 200 allows for the wire 168-1 to be routed from a distal end 174 of the coupler 170, thereby reducing a potential lever arm and force applied to the wires up until the point at which they reach an optimal location near the neutral axis DD, for example, where they exit the proximal end 172 of the coupler 170. In some embodiments, the alternate wire routing channel 200 can connect with the sensor channel 178-1, such that wires can be routed from the alternate wire routing channel 200 out a proximal end of the sensor channel 178-1. However, in some embodiments, the alternate wire routing channel 200 can be routed out the proximal end 172 of the coupler 170 separately from the sensor channel 178-1. For example, although not depicted, the alternate wire routing channel 200 can define a separate channel in the proximal end 172 of the coupler 170 from the sensor channel 178-1. In some embodiments, the wire 168-1 can be secured within the alternate wire routing channel 200 via an adhesive, for example.

As further depicted with respect to Fig. 4, in some embodiments, the proximal end of the base portion 196 can be removed adjacent the proximal end 172 of the coupler. For example, the proximal end 172 can define recessed areas that extend distally along each one of the sensor grooves 178-1, 178-2, thus forming the cutouts 194-1, 194-2. This can allow for wires associated with the respective sensors disposed in the first and second sensor grooves 178-1, 178-2 to pass through the cutouts 194-1, 194-2 and be disposed closer to a neutral axis DD of the coupler 170.

In some embodiments, a proximal end of the base portion 196 can include beveled proximal edges 198 to better enable a transition for wires contained in each one of the first and second sensor grooves 178-1, 178-2 through the cutouts 194-1, 194-2 towards the neutral axis DD. In an example, the beveled proximal edges 198-1, 198-2 of the proximal ends of the base portion 196 can be beveled towards the neutral axis AA in a distal to proximal direction. In some embodiments, the beveled proximal edges 198-1, 198-2 can be a straight bevel, which is depicted. However, in some embodiments, the beveled proximal edges 198-1, 198-2 can include a radiused edge to provide a smooth transition for the wires towards the neutral axis DD, as further discussed herein.

In some embodiments where the alternate wire routing channel 200 defines separate channels in the proximal end 172 of the coupler 170 from the sensor channel 178-1, the proximal ends of the separate channels can include beveled proximal edges, as discussed herein. The beveled proximal edges can allow for the wires traveling through the alternate wire routing channels to better transition towards the neutral axis DD.

Fig. 5 depicts a circular mapping catheter, in accordance with embodiments of the present disclosure. Referring now to Fig. 5 there is shown one embodiment of a bidirectional catheter 212 including catheter handle 214 and catheter shaft 216, including loop member 218. Bidirectional catheter 212 additionally includes sliding mechanism 220 located in track 222. Sliding mechanism 220 controls the diameter adjustment of loop member 218, as further discussed in WO 2017/177121. Sliding mechanism 220 will generally include teeth or gears (not shown in Figure 10) to suitably engage a gear assembly or member located in the catheter handle, as further described in WO 2017/177121. In some embodiments, the loop member 218 and/or the catheter shaft 216 can include a wire management coupler, as discussed herein, with respect to embodiments of the present disclosure.

Embodiments are described herein of various apparatuses, systems, and/or methods. Numerous specific details are set forth to provide a thorough understanding of the overall structure, function, manufacture, and use of the embodiments as described in the specification and illustrated in the accompanying drawings. It will be understood by those skilled in the art, however, that the embodiments may be practiced without such specific details. In other instances, well-known operations, components, and elements have not been described in detail so as not to obscure the embodiments described in the specification. Those of ordinary skill in the art will understand that the embodiments described and illustrated herein are non-limiting examples, and thus it may be appreciated that the specific structural and functional details disclosed herein may be representative and do not necessarily limit the scope of the embodiments, the scope of which is defined solely by the appended claims.

Reference throughout the specification to "various embodiments," "some embodiments," "one embodiment," or "an embodiment", or the like, means that a particular feature, structure, or characteristic described in connection with the embodiment(s) is included in at least one embodiment. Thus, appearances of the phrases "in various embodiments," "in some embodiments," "in one embodiment," or "in an embodiment," or the like, in places throughout the specification, are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features, structures, or characteristics of one or more other embodiments without limitation given that such combination is not illogical or non-functional.

It will be appreciated that the terms "proximal" and "distal" may be used throughout the specification with reference to a clinician manipulating one end of an instrument used to treat a patient. The term "proximal" refers to the portion of the instrument closest to the clinician and the term "distal" refers to the portion located furthest from the clinician. It will be further appreciated that for conciseness and clarity, spatial terms such as "vertical," "horizontal," "up," and "down" may be used herein with respect to the illustrated embodiments. However, surgical instruments may be used in many orientations and positions, and these terms are not intended to be limiting and absolute.

All directional references (e.g., upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the devices. Joinder references (e.g., affixed, attached, coupled, connected, and the like) are to be construed broadly and may include intermediate members between a connection of elements and relative movement between elements. As such, joinder references do not necessarily infer that two elements are directly connected and in fixed relationship to each other. It is intended that all matter contained in the above description or shown in the accompanying drawings shall be interpreted as illustrative only and not limiting. Changes in detail or structure may be made without departing from the spirit of the disclosure as defined in the appended claims.

## Claims

1. A catheter, comprising:
an elongate shaft (107) including a proximal end (122) and a distal end (124), the elongate shaft (107) defining a shaft longitudinal axis;
a coupler (120) disposed within a distal end of the elongate shaft, wherein the coupler (120) defines:
a first sensor groove (128-1) and a second sensor groove (128-2) in an exterior surface of the coupler (120) and a coupler longitudinal axis; and
a first and second wire management feature located at a proximal end of each one of the first sensor groove (128-1) and the second sensor groove (128-2), **characterized in that**
the first and second wire management features include a first and second recessed area (144-1, 144-2) defined in a proximal end (122) of the coupler (120), at a proximal end of the first and second sensor grooves, wherein the first and second wire management features include beveled proximal edges (148-1, 148-2) of each one of the first and second sensor grooves (128-1, 128-2) including proximal edges of the first and second sensor grooves (128-1, 128-2) that are beveled towards the coupler longitudinal axis in a distal to proximal direction.

2. The catheter of claim 1, wherein the recessed area (144-1, 144-2) extends distally from the proximal end (122) of the coupler (120), along each one of the first and second sensor grooves (128-1, 128-2).

3. The catheter of claim 2, wherein a first five degree of freedom magnetic position sensor (132-1) is disposed in the first sensor groove (128-1) and a second five degree of freedom magnetic position sensor (132-2) disposed in the second sensor groove (128-2).

4. The catheter of claim 3, wherein each of the first and second five degree of freedom magnetic position sensors (132-1, 132-2) include a set of connecting wires (118-1, 118-2) that extend proximally from each of the first and second five degree of freedom magnetic position sensors (132-1, 132-2).

5. The catheter of claim 4, wherein each set of connecting wires (118-1, 118-2) extend from the distal ends of each one of the five degree of freedom magnetic position sensors (132-1, 132-2) toward the coupler longitudinal axis and through each one of the recessed areas (144-1, 144-2).

6. The catheter of claim 5, further comprising a heat shrink tubing through which the connecting wires (118-1, 118-2) extend, wherein a distal end of the heat shrink tubing is attached to the proximal end (122) of the coupler (120).

7. The catheter of claim 1, further comprising first and second five degree of freedom magnetic position sensors (132-1, 132-2) disposed in each one of the first sensor groove (128-1) and second sensor groove (128-2), the first and second five degree of freedom magnetic position sensors (132-1, 132-2) each including connecting wires (118-1, 118-2) that extend proximally from each of the first and second five degree of freedom magnetic position sensors (132-1, 132-2).

8. The catheter of claim 7, wherein the connecting wires (118-1, 118-2) extend proximally towards the coupler longitudinal axis, adjacent to the beveled proximal edges (148-1, 148-2).

9. The catheter of claim 8, further comprising a heat shrink tubing through which the connecting wires (118-1, 118-2) extend, wherein a distal end of the heat shrink tubing is disposed proximally with respect to the beveled proximal edges (148-1, 148-2).

## Patentansprüche

1. Katheter mit:
einem länglichen Schaft (107), der ein proximales Ende (122) und ein distales Ende (124) aufweist, wobei der längliche Schaft (107) eine Schaftlängsachse definiert;
einem Koppler (120), der sich in einem distalen Ende des länglichen Schafts befindet, wobei der Koppler (120) definiert:
eine erste Sensorrille (128-1) und eine zweite Sensorrille (128-2) in einer äußeren Fläche des Kopplers (120), und eine Kopplerlängsachse; und
ein erstes und zweites Kabelmanagementmerkmal, die sich an einem proximalen Ende von jeder von der ersten Sensorrille (128-1) und der zweiten Sensorrille (128-2) befinden, **dadurch gekennzeichnet, dass**
das erste und das zweite Kabelmanagementmerkmal einen ersten und zweiten vertieften Bereich (144-1, 144-2) aufweisen, die in einem proximalen Ende (122) des Kopplers (120) an einem proximalen Ende der ersten und zweiten Sensorrille definiert sind, wobei das erste und zweite Kabelmanagementmerkmal abgeschrägte proximale Ränder (148- 1, 148-2) von jeder von der ersten und zweiten Sensorrille (128-1, 128-2) aufweisen, einschließlich proximaler Ränder der ersten und der zweiten Sensorrille (128-1, 128-2), die in Richtung der Kopplerlängsachse in einer distal-zu-proximal Richtung abgeschrägt sind.

2. Katheter nach Anspruch 1, bei dem der vertiefte Bereich (144-1, 144-2) sich distal von dem proximalen Ende (122) des Kopplers (120) entlang jeder von der ersten und zweiten Sensorrille (128-1, 128-2) erstreckt.

3. Katheter nach Anspruch 2, bei dem ein erster Magnetpositionssensor (132-1) fünften Freiheitsgrades sich in der ersten Sensorrille (128-1) befindet, und ein zweiter Magnetpositionssensor (132-2) fünften Freiheitsgrades sich in der zweiten Sensorrille (128-2) befindet.

4. Katheter nach Anspruch 3, bei dem jeder von dem ersten und zweiten Magnetpositionssensor (132-1, 132-2) fünften Freiheitsgrades einen Satz von Verbindungsdrähten (118-1, 118-2) aufweist, die sich proximal von jedem von dem ersten und zweiten Magnetpositionssensor (132-1, 132-2) fünften Freiheitsgrades aus erstrecken.

5. Katheter nach Anspruch 4, bei dem jeder Satz von Verbindungsdrähten (118-1, 118-2) sich von den distalen Enden von jedem von den Magnetpositionssensoren (132-1, 132-2) fünften Freiheitsgrades in Richtung der Kopplerlängsachse und durch jeden der vertieften Bereiche (144-1, 144-2) erstreckt.

6. Katheter nach Anspruch 5, ferner mit einem Wärmeschrumpfschlauch, durch den sich die Verbindungsdrähte (118-1, 118-2) erstrecken, wobei ein distales Ende des Wärmeschrumpfschlauchs an dem proximalen Ende (122) des Kopplers (120) angebracht ist.

7. Katheter nach Anspruch 1, ferner mit einem ersten und zweiten Magnetpositionssensor (132-1, 132-2) fünften Freiheitsgrades, die sich in jeder von der ersten Sensorrille (128-1) und der zweiten Sensorrille (128-2) befinden, wobei der erste und zweite Magnetpositionssensor (132-1, 132-2) fünften Freiheitsgrades jeweils Verbindungsdrähte (118-1, 118-2) aufweisen, die sich von jedem von dem ersten und zweiten Magnetpositionssensor (132-1, 132-2) fünften Freiheitsgrades aus proximal erstrecken.

8. Katheter nach Anspruch 7, bei dem die Verbindungsdrähte (118-1, 118-2) sich proximal in Richtung der Kopplerlängsachse benachbart zu den abgeschrägten proximalen Rändern (148-1, 148-2) erstrecken.

9. Katheter nach Anspruch 8, ferner mit einem Wärmeschrumpfschlauch, durch den sich die Verbindungsdrähte (118-1, 118-2) erstrecken, wobei ein distales Ende des Wärmeschrumpfschlauchs proximal bezüglich der abgeschrägten proximalen Ränder (148-1, 148-2) vorgesehen ist.

## Revendications

1. Cathéter comprenant :
une tige allongée (107) comprenant une extrémité proximale (122) et une extrémité distale (124), la tige allongée (107) définissant un axe longitudinal de tige ;
un coupleur (120) disposé à l'intérieur d'une extrémité distale de la tige allongée, dans lequel le coupleur (120) définit :
une première rainure de capteur (128-1) et une seconde rainure de capteur (128-2) dans une surface extérieure du coupleur (120) et un axe longitudinal de coupleur ; et
un premier et un second élément de gestion de fil situés à une extrémité proximale de chacune de la première rainure de capteur (128-1) et de la seconde rainure de capteur (128-2), **caractérisé en ce que**
les premier et second éléments de gestion de fil comprennent une première et une seconde zone en retrait (144-1, 144-2) définie dans une extrémité proximale (122) du coupleur (120), à une extrémité proximale des première et seconde rainures de capteur, dans lequel les premier et second éléments de gestion de fil comprennent des bords proximaux biseautés (148-1, 148-2) de chacune des première et seconde rainures de capteur (128-1, 128-2), y compris les bords proximaux des première et seconde rainures de capteur (128-1, 128-2), qui sont biseautés vers l'axe longitudinal de coupleur dans une direction distale à proximale.

2. Cathéter de la revendication 1, dans lequel la zone en retrait (144-1, 144-2) s'étend distalement à partir de l'extrémité proximale (122) du coupleur (120), le long de chacune de la première et la seconde rainures de capteur (128-1, 128-2).

3. Cathéter de la revendication 2, dans lequel un premier capteur de position magnétique à cinq degrés de liberté (132-1) est disposé dans la première rainure de capteur (128-1) et un second capteur de position magnétique à cinq degrés de liberté (132-2) est disposé dans la seconde rainure de capteur (128-2).

4. Cathéter de la revendication 3, dans lequel chacun des premiers et seconds capteurs de position magnétique à cinq degrés de liberté (132-1, 132-2) comprend un ensemble de fils de connexion (118-1, 118-2) qui s'étendent de manière proximale à partir de chacun des premiers et seconds capteurs de position magnétique à cinq degrés de liberté (132-1, 132-2).

5. Cathéter de la revendication 4, dans lequel chaque ensemble de fils de connexion (118-1, 118-2) s'étend des extrémités distales de chacun des capteurs de position magnétique à cinq degrés de liberté (132-1, 132-2) vers l'axe longitudinal de coupleur et à travers chacune des zones en retrait (144-1, 144-2).

6. Cathéter de la revendication 5, comprenant en outre une gaine thermorétractable à travers laquelle les fils de connexion (118-1, 118-2) s'étendent, dans lequel une extrémité distale de la gaine thermorétractable est fixée à l'extrémité proximale (122) du coupleur (120).

7. Cathéter de la revendication 1, comprenant en outre un premier et un second capteurs de position magnétique à cinq degrés de liberté (132-1, 132-2) disposés dans la première rainure de capteur (128-1) et la seconde rainure de capteur (128-2), le premier et le second capteurs de position magnétique à cinq degrés de liberté (132-1, 132-2) comprenant chacun des fils de connexion (118-1, 118-2) qui s'étendent de manière proximale à partir de chacun des premier et second capteurs de position magnétique à cinq degrés de liberté (132-1, 132-2).

8. Cathéter de la revendication 7, dans lequel les fils de connexion (118-1, 118-2) s'étendent de manière proximale vers l'axe longitudinal de coupleur, adjacent aux bords proximaux biseautés (148-1, 148-2).

9. Cathéter de la revendication 8, comprenant en outre une gaine thermorétractable à travers laquelle les fils de connexion (118-1, 118-2) s'étendent, dans lequel une extrémité distale de la gaine thermorétractable est disposée de manière proximale par rapport aux bords proximaux biseautés (148-1, 148-2).
